# EUROPEAN PATENT APPLICATION

(11) **EP 4 590 078 A1**
(43) Date of publication of application: **23.07.2025**
(21) Application number: 25152199.3
(22) Date of filing: 16.01.2025
(51) Int. Cl.: H05K 1/11, H05K 1/18, H05K 3/24, H05K 3/40

(54) **ELECTRONIC DEVICES AND METHODS FOR FORMING CONTACTS ON ELECTRONIC DEVICES**

(30) Priority: 17.01.2024 US 202463621931 P; 07.01.2025 US 202519012580
(71) Applicant: Meta Platforms Technologies, LLC, Menlo Park, CA 94025 (US)
(72) Inventor: BAO, Jianer, Menlo Park, 94025 (US); JI, Xiangying, Menlo Park, 94025 (US); LI, Jingye, Menlo Park, 94025 (US)
(74) Representative: Murgitroyd & Company

(57) **Abstract**

Electronic devices include a non-conductive housing component (102), a contact (104) comprising a conductive seed material (310) formed over an external surface of the non-conductive housing component (102) and a bulk conductive contact material (312) formed over the conductive seed material (310), and an electronic component connected to the bulk conductive contact material (312) and the conductive seed material (310). Manufacturing methods are also disclosed.

## Description

### TECHNICAL FIELD

The present disclosure generally relates to forming sensor (e.g., EMG) contacts, charging contacts, antennas, and/or other conductive elements on consumer electronic devices (e.g., small consumer electronic devices, wearable devices, etc.).

### BACKGROUND

Many small consumer electronic devices (e.g., watches, phones, fitness trackers, wireless earbuds, smart glasses, artificial-reality glasses, and the like) include rechargeable batteries. These batteries can be charged using exposed charging contacts on the electronic device. Similarly, some sensing devices (e.g., fitness trackers, electromyography (EMG) sensors, etc.) include external electrodes that can be used to sense electrical signals generated by a user's body. Small consumer electronic devices may also include antennas for wireless transmission of data.

Charging contacts, electrodes, and antennas can be formed on electronic devices by performing a process of overmolding a contact material on or in a housing material and/or machining (e.g., multi-axis machining) to achieve a small form factor and acceptable quality. Such processes can sometimes be time-consuming, difficult to implement, and expensive.

### SUMMARY

According to a first aspect, there is provided an electronic device, comprising: a non-conductive housing component; a conductive seed material formed over an external surface of the non-conductive housing component; a bulk conductive contact material formed over the conductive seed material; and an electronic component connected to the bulk conductive contact material and the conductive seed material.

The electronic device may further comprise a conductive via passing through the non-conductive housing component and connecting the bulk conductive contact material and the conductive seed material to the electronic component.

The electronic device may further comprise a conductive connection, comprising at least one of: a conductive post through the non-conductive housing component; a conductive plating on a surface of the non-conductive housing component; or a conductive strip molded in the non-conductive housing component, the conductive connection connecting the bulk conductive contact material and the conductive seed material to the electronic component.

The electronic component may comprise a battery and the bulk conductive contact material may comprise charging contacts for the battery.

The electronic device may further comprise at least one conductive coating material over at least a portion of the bulk conductive contact material. The at least one conductive coating material may comprise at least one of: nitride; diamond-like carbon; chromium; palladium; gold; platinum; silver; or rhodium.

The electronic component may comprise a sensing circuit and the bulk conductive contact material may comprise at least one sensor electrode for the sensing circuit.

The sensing circuit may comprise an electromyography sensing circuit.

The bulk conductive contact material may comprise an electrode pair including two electrodes.

According to a second aspect, there is provided a method of forming contacts for an electronic device, the method comprising: forming a conductive seed material over a non-conductive housing component of the electronic device; forming a bulk conductive contact material over the conductive seed material; and connecting the bulk conductive contact material and the conductive seed material to an electronic component.

Connecting the bulk conductive contact material and the conductive seed material to the electronic component may comprise: forming a conductive via through at least a portion of the non-conductive housing component; forming the conductive seed material in electrical contact with the conductive via; and electrically connecting the conductive via to the electronic component.

Connecting the bulk conductive contact material and the conductive seed material to the electronic component may comprise: molding at least one of a conductive post or a conductive strip in the non-conductive housing component; forming the conductive seed material in electrical contact with the conductive post or the conductive strip; and electrically connecting the conductive post or the conductive strip to the electronic component.

The electronic component may comprise a battery.

The electronic component may comprise a sensing circuit.

The sensing circuit may comprise an electromyography sensing circuit.

The method may further comprise coating at least a portion of the bulk conductive contact material with at least one conductive coating material. The at least one conductive coating material may comprise at least one of: nitride; diamond-like carbon; chromium; palladium; gold; platinum; silver; or rhodium.

Forming the conductive seed material over the non-conductive housing component may comprise forming at least one of: copper; nickel, titanium, chromium; or gold over the non-conductive housing component.

The method may further comprise etching a surface of the non-conductive housing component. Forming the conductive seed material may comprise forming the conductive seed material on the etched surface of the non-conductive housing component.

Forming the bulk conductive contact material over the conductive seed material may comprise forming at least one of: copper; nickel; or silver over the conductive seed material.

Forming the bulk conductive contact material over the conductive seed material may comprise forming the bulk conductive contact material to a thickness between 1 µm and 30 µm.

### BRIEF DESCRIPTION OF DRAWINGS

The accompanying drawings illustrate a number of examples and are a part of the specification. Together with the following description, these drawings demonstrate and explain various principles of the present disclosure.
FIG. 1 is a side cross-sectional view of an electronic device.
FIG. 2 is a side cross-sectional view of an electronic device.
FIG. 3 is a detailed cross-sectional view of a contact of an electronic device.
FIG. 4 is a flow diagram illustrating a method of forming contacts for an electronic device.
FIG. 5 is an illustration of example augmented-reality glasses that may be used in connection with embodiments of this disclosure.
FIG. 6 is an illustration of an example virtual-reality headset that may be used in connection with embodiments of this disclosure.
FIGS. 7A and 7B are illustrations of an example human-machine interface configured to be worn around a user's lower arm or wrist.
FIGS. 8A and 8B are illustrations of an example schematic diagram with internal components of a wearable system.

Throughout the drawings, identical reference characters and descriptions indicate similar, but not necessarily identical, elements. While the examples described herein are susceptible to various modifications and alternative forms, specific arrangements have been shown by way of example in the drawings and will be described in detail herein. However, the examples described herein are not intended to be limited to the particular forms disclosed. Rather, the present disclosure covers all modifications, equivalents, and alternatives falling within this disclosure.

### DETAILED DESCRIPTION

The present disclosure generally relates to forming sensor (e.g., EMG) contacts, charging contacts, antennas, and/or other conductive elements on consumer electronic devices (e.g., small consumer electronic devices, wearable devices, etc.). Such contacts or antennas are generally referred to herein as "contacts" for simplicity. The contacts may be formed by directly plating or depositing a conductive material on an underlying non-conductive housing or other substrate material, including plastics, ceramics, glass, or other non-conductive substrates that may be part of the device.

The disclosed processes can include etching (e.g., solvent etching, laser etching, plasma etching, etc.) the substrate to create a rough surface for good adhesion. Next, a conductive seed layer may be deposited on the surface, which can be accomplished by metal colloidal activation plus electroless plating and/or by physical vapor deposition (PVD) coating. The seed layer may be patterned, such as by masking, using an electrophoretic paint and laser carving out the area to be deposited, and/or by using direct laser activation. A bulk conductive material (e.g., copper (Cu), nickel (Ni), silver (Ag), chromium (Cr), etc.) can then be electroplated or deposited on the patterned seed layer. A conductive protective coating (e.g., of palladium (Pd), gold (Au), platinum (Pt), silver (Ag), rhodium (Rh), etc.) can also be applied to the bulk conductive material. For high wear-and-tear applications, a coating of conductive nitrides or conductive diamond-like carbon (DLC) could also be used. In some examples, the plated/coated contact material can be connected to underlying electronics with a conductive via and/or conductive post or by continuous plating from the front side to the back side of the housing. Due to the potential thinness of the contacts, some examples of the present disclosure are suitable for low-current signal transmission, such as charging and/or sensing for small wearable devices (e.g., watches, exercise sensors, wearable EMG sensors, etc.).

In some examples, the term "contacts" may refer to any conductive contacts, including charging contacts, sensor electrodes, antennas, and the like. The terms "conductive" and "non-conductive" may generally refer to electrically conductive and electrically non-conductive. These terms can be used relative to each other, meaning that a conductive material may be more electrically conductive (e.g., having a lower resistivity) than a corresponding non-conductive material, and a non-conductive material may be less electrically conductive (e.g., having a higher resistivity) than a corresponding conductive material.

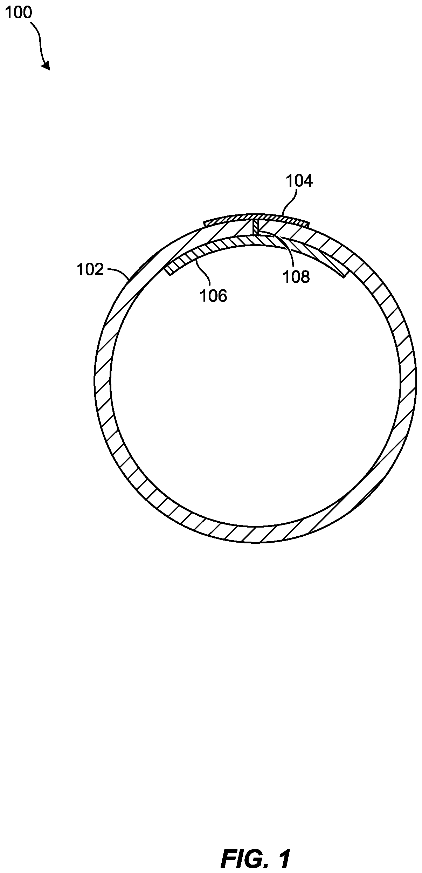

FIG. 1 is a side cross-sectional view of an electronic device 100. The electronic device 100 may include a non-conductive housing component 102, at least one contact 104 formed on an external surface of the non-conductive housing component 102, and an electronic component 106 connected (e.g., electrically connected) to the contact 104. The electronic device 100 is illustrated in the form of a watch or wristband, but the disclosure is not so limited. Additional examples of electronic devices according to the present disclosure may include a ring, an armband, an earbud, augmented-reality glasses, a fitness tracker, or any other electronic device that may include external, exposed contacts on a non-conductive housing.

In some examples, the non-conductive housing component 102 may be formed of, or may include, a non-conductive material, such as a polymer material, a ceramic material, a glass material, a composite material, etc.

The contact 104 may be, for example, a charging contact or a sensor electrode. The electronic component 106 may include, for example, a battery, a microcontroller, a communication element (e.g., for wireless communication and/or wired communication), a sensing circuit (e.g., an EMG sensing circuit), a display screen, and/or a touch screen, etc. In examples in which the electronic component 106 includes a battery, the contact 104 may be a charging contact. In cases where the electronic component 106 includes a sensing circuit, the contact 104 may be a sensor electrode. The electronic component 106 may be positioned on an external surface of the non-conductive housing component 102 (e.g., as illustrated in FIG. 1), encased within the non-conductive housing component 102, and/or partially within the non-conductive housing component 102.

In some examples, the contact 104 may include a relatively thin material formed on a surface of the non-conductive housing component 102. For example, the contact 104 may have a thickness over the non-conductive housing component 102 within a range of 1 µm to 30 µm. The contact 104 may have a sheet resistance ranging from less than 1 milliohm to about 200 milliohms, which may enable sufficient electrical current to pass therethrough for sensing and/or charging.

The contact 104 may be connected to the electronic component 106 in a variety of ways. For example, a conductive connection 108, such as a conductive via, post, and/or strip, may pass along or through the non-conductive housing component 102 to connect the contact 104 to the electronic component 106. If the conductive connection 108 includes a conductive via, the via may pass through the non-conductive housing component 102 by coating and/or filling a hole through the non-conductive housing component with a conductive material. If the conductive connection 108 includes a conductive post or strip, the post or strip could be formed by molding the conductive post or strip in or on the non-conductive housing component 102. In additional examples, the housing component 102 may include more than one piece, and conductive plating can be applied around an edge and back side of at least one of the pieces of the housing component 102 to reach the electronic component 106. In further examples, a conductive adhesive material may be used to connect the electronic component 106 to the contact 104.

FIG. 1 illustrates the contact 104 on an outer external surface (e.g., a radially outer external surface) of the non-conductive housing component 102, and the electronic component 106 on an inner external surface (e.g., a radially inner external surface) of the non-conductive housing component 102. However, the present disclosure is not so limited and other configurations are possible. For example, the contact 104 may include one or more contacts on an inner external surface of the non-conductive housing component 102 and the electronic component could be on an outer external surface or at least partially within an inner compartment of the non-conductive housing component.

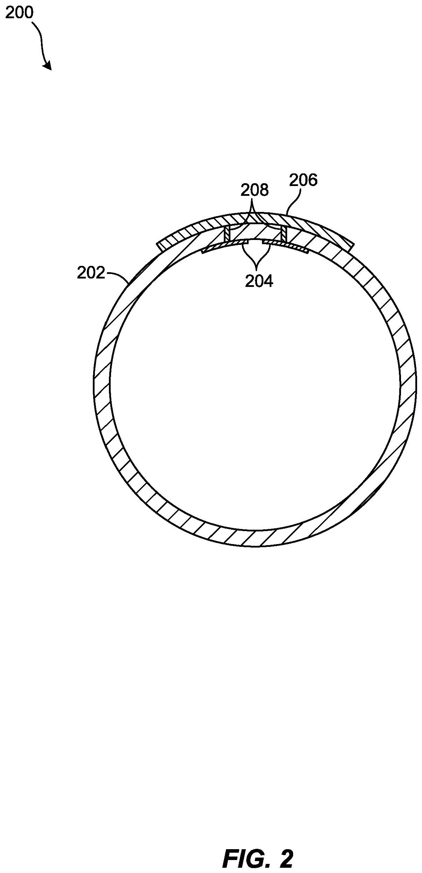

For example, FIG. 2 is a side cross-sectional view of an electronic device 200, which may include a non-conductive housing component 202, two contacts 204 on an inner (e.g., radially inner) external surface of the non-conductive housing component 202, and an electronic component 206 on an outer external surface of the non-conductive housing component 202. The two contacts 204 may be connected to the electronic component 206 with respective electrical connections 208, such as conductive vias, posts, strips, or combinations thereof.

In some implementations, the positioning of the contacts 204 on the inner external surface of the non-conductive housing component 206 may be suitable for using the contacts 204 as sensors, such as EMG sensors or the like, since the contacts 204 may be located against a user's skin when the electronic device 200 is worn by a user.

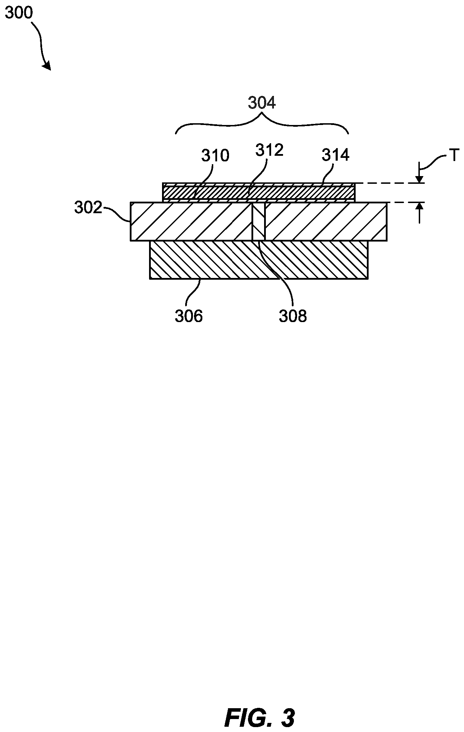

FIG. 3 is a detailed cross-sectional view of a contact 304 of an electronic device 300. The electronic device 300 may include a non-conductive housing component 302, one or more contacts 304, and an electronic device 306 (e.g., a battery, a sensing circuit, etc.) connected to the one or more contacts 304, such as with a conductive via, post, and/or strip.

The contact 304 may include a conductive seed material 310 formed on a surface of the non-conductive housing component 302. The conductive seed material 310 could be deposited through metal colloidal activation plus electroless plating, or through physical vapor deposition (PVD) coating. For example, electroless copper could be deposited on metal colloidal activated plastics. In another example, conductive PVD metals (such as chromium, nickel, titanium, copper, gold, etc.) could be deposited on ceramics, glass, or some plastics.

A pattern of the conductive seed material 310 could be achieved through a variety of ways, such as by masking, by using electrophoretic paint and laser carving out an area to be deposited, and/or by directly using laser activation. To improve adhesion between the non-conductive housing component 302 and conductive seed material 310, the surface of the non-conductive housing component 302 can be etched to create increased roughness and/or an activated surface. By way of example, suitable etching techniques may include solvent etching, laser etching, and/or plasma etching. After a conductive seed material 310 is deposited, a strike plating may be used to improve a uniformity and increase conductivity of the contact 304, followed by additional electroplating of a bulk conductive contact material 312 (e.g., copper, nickel, silver, etc.) to a predetermined contact thickness T over the non-conductive housing component 302. By way of example and not limitation, to reduce stress but provide sufficient material for electrical current to pass through, in some examples the thickness T of the contact 304 may be between about 1 µm and about 30 µm. A rack plating process may be used to form at least a portion of the contact 304, but other plating process could be possible for certain form factors.

In some examples, a coating material 314 may be deposited over the bulk conductive contact material 312. The coating material 314 may include a conductive noble metal material (e.g., palladium, gold, platinum, silver, or rhodium), such as for protection against oxidation and corrosion. In additional examples, the coating material 314 may include a conductive mechanical protection material to protect against scratches and dents, such as a conductive nitride and/or a conductive diamond-like carbon (DLC) material.

If the contact 304 is used for charging a battery, the contact 304 may be configured to conduct electrical currents in a milliamp range (e.g., 1 mA to several hundred mA). If the contact 304 is used as a sensing contact, the contact 304 may be configured to conduct electrical currents in a microamp range (e.g., 1 µA to several hundred µA).

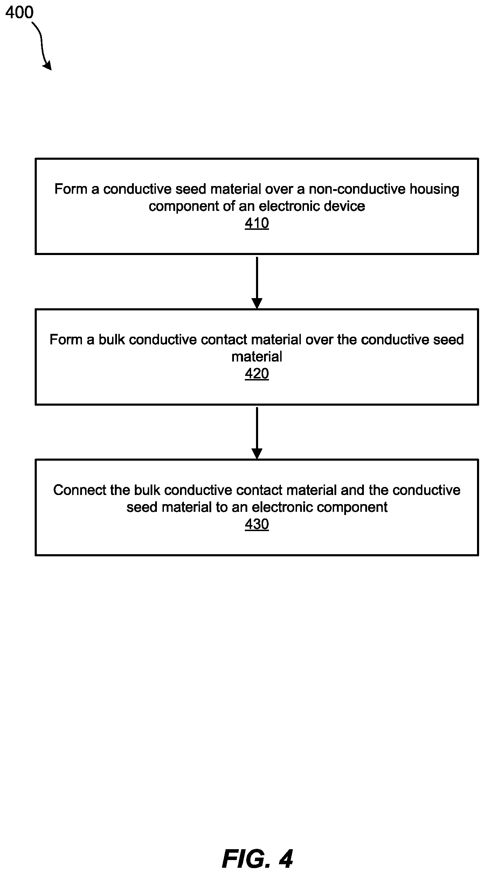

FIG. 4 is a flow diagram illustrating a method 400 of forming contacts for an electronic device. At operation 410, a conductive seed material may be formed over a non-conductive housing component of the electronic device. Operation 410 may be performed in a variety of ways, such as any of the ways discussed above with reference to FIGS. 1-3.

At operation 420, a bulk conductive contact material may be formed over the conductive seed material. Operation 420 may be performed in a variety of ways, such as any of the ways discussed above with reference to FIGS. 1-3.

At operation 430, the bulk conductive contact material and the conductive seed material may be connected to an electronic component, such as a battery or a sensing circuit. Operation 430 may be performed in a variety of ways, such as any of the ways discussed above with reference to FIGS. 1-3.

In some examples, the method 400 may include additional operations, such as any of the operations discussed above with reference to FIGS. 1-3. For example, the method 400 may include etching the non-conductive housing component prior to forming the conductive seed material, and/or positioning the conductive seed material and the bulk conductive contact material on an inner external surface or on an outer external surface of the non-conductive housing component forming a coating material over the bulk conductive contact material, etc.

Accordingly, the present disclosure includes electronic devices and methods of forming contacts for electronic devices that may improve manufacturability, reliability, functionality, and cost compared to prior known devices and methods.

Embodiments of the present disclosure may include or be implemented in -conjunction with various types of artificial-reality systems. Artificial reality is a form of reality that has been adjusted in some manner before presentation to a user, which may include, for example, a virtual reality, an augmented reality, a mixed reality, a hybrid reality, or some combination and/or derivative thereof. Artificial-reality content may include completely computer-generated content or computer-generated content combined with captured (e.g., real-world) content. The artificial-reality content may include video, audio, haptic feedback, or some combination thereof, any of which may be presented in a single channel or in multiple channels (such as stereo video that produces a three-dimensional (3D) effect to the viewer). Additionally, in some examples, artificial reality may also be associated with applications, products, accessories, services, or some combination thereof, that are used to, for example, create content in an artificial reality and/or are otherwise used in (e.g., to perform activities in) an artificial reality.

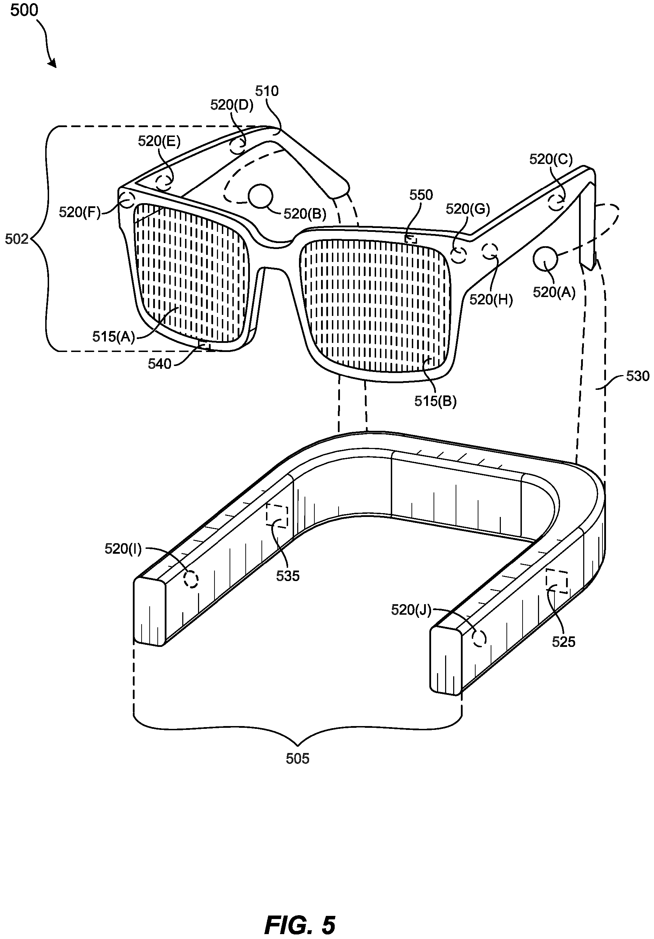

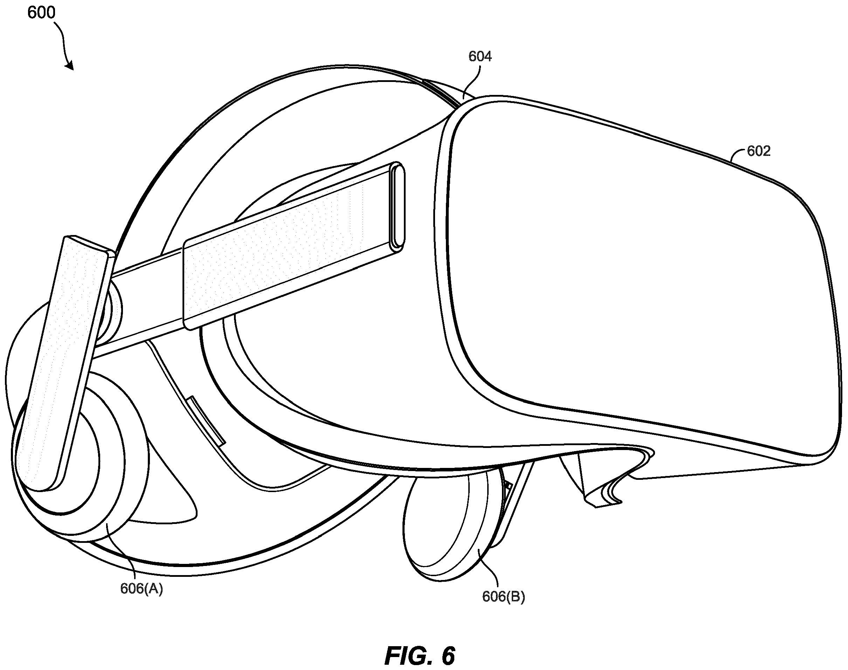

Artificial-reality systems may be implemented in a variety of different form factors and configurations. Some artificial-reality systems may be designed to work without near-eye displays (NEDs). Other artificial-reality systems may include an NED that also provides visibility into the real world (such as, e.g., augmented-reality system 500 in FIG. 5) or that visually immerses a user in an artificial reality (such as, e.g., virtual-reality system 600 in FIG. 6). While some artificial-reality devices may be self-contained systems, other artificial-reality devices may communicate and/or coordinate with external devices to provide an artificial-reality experience to a user. Examples of such external devices include handheld controllers, mobile devices, desktop computers, devices worn by a user, devices worn by one or more other users, and/or any other suitable external system.

Turning to FIG. 5, an augmented-reality system 500 may include an eyewear device 502 with a frame 510 configured to hold a left display device 515(A) and a right display device 515(B) in front of a user's eyes. The display devices 515(A) and 515(B) may act together or independently to present an image or series of images to a user. While the augmented-reality system 500 includes two displays, examples of this disclosure may be implemented in augmented-reality systems with a single NED or more than two NEDs.

In some examples, the augmented-reality system 500 may include one or more sensors, such as a sensor 540. The sensor 540 may generate measurement signals in response to motion of the augmented-reality system 500 and may be located on substantially any portion of the frame 510. The sensor 540 may represent one or more of a variety of different sensing mechanisms, such as a position sensor, an inertial measurement unit (IMU), a depth camera assembly, a structured light emitter and/or detector, or any combination thereof. In some examples, the augmented-reality system 500 may or may not include the sensor 540 or may include more than one sensor. In examples in which the sensor 540 includes an IMU, the IMU may generate calibration data based on measurement signals from the sensor 540. Examples of the sensor 540 may include, without limitation, accelerometers, gyroscopes, magnetometers, other suitable types of sensors that detect motion, sensors used for error correction of the IMU, or some combination thereof.

In some examples, the augmented-reality system 500 may also include a microphone array with a plurality of acoustic transducers 520(A)-520(J), referred to collectively as acoustic transducers 520. The acoustic transducers 520 may represent transducers that detect air pressure variations induced by sound waves. Each acoustic transducer 520 may be configured to detect sound and convert the detected sound into an electronic format (e.g., an analog or digital format). The microphone array in FIG. 5 may include, for example, ten acoustic transducers: 520(A) and 520(B), which may be designed to be placed inside a corresponding ear of the user, acoustic transducers 520(C), 520(D), 520(E), 520(F), 520(G), and 520(H), which may be positioned at various locations on the frame 510, and/or acoustic transducers 520(I) and 520(J), which may be positioned on a corresponding neckband 505.

In some examples, one or more of the acoustic transducers 520(A)-(J) may be used as output transducers (e.g., speakers). For example, the acoustic transducers 520(A) and/or 520(B) may be earbuds or any other suitable type of headphone or speaker.

The configuration of the acoustic transducers 520 of the microphone array may vary. While the augmented-reality system 500 is shown in FIG. 5 as having ten acoustic transducers 520, the number of acoustic transducers 520 may be greater or less than ten. In some examples, using higher numbers of acoustic transducers 520 may increase the amount of audio information collected and/or the sensitivity and accuracy of the audio information. In contrast, using a lower number of acoustic transducers 520 may decrease the computing power required by an associated controller 550 to process the collected audio information. In addition, the position of each acoustic transducer 520 of the microphone array may vary. For example, the position of an acoustic transducer 520 may include a defined position on the user, a defined coordinate on the frame 510, an orientation associated with each acoustic transducer 520, or some combination thereof.

The acoustic transducers 520(A) and 520(B) may be positioned on different parts of the user's ear, such as behind the pinna, behind the tragus, and/or within the auricle or fossa. Or there may be additional acoustic transducers 520 on or surrounding the ear in addition to the acoustic transducers 520 inside the ear canal. Having an acoustic transducer 520 positioned next to an ear canal of a user may enable the microphone array to collect information on how sounds arrive at the ear canal. By positioning at least two of the acoustic transducers 520 on either side of a user's head (e.g., as binaural microphones), the augmented-reality device 500 may simulate binaural hearing and capture a 3D stereo sound field around about a user's head. In some examples, the acoustic transducers 520(A) and 520(B) may be connected to the augmented-reality system 500 via a wired connection 530, and in other examples the acoustic transducers 520(A) and 520(B) may be connected to the augmented-reality system 500 via a wireless connection (e.g., a BLUETOOTH connection). In still other examples, the acoustic transducers 520(A) and 520(B) may not be used at all in conjunction with the augmented-reality system 500.

The acoustic transducers 520 on the frame 510 may be positioned in a variety of different ways, including along the length of the temples, across the bridge, above or below the display devices 515(A) and 515(B), or some combination thereof. The acoustic transducers 520 may also be oriented such that the microphone array is able to detect sounds in a wide range of directions surrounding the user wearing the augmented-reality system 500. In some examples, an optimization process may be performed during manufacturing of the augmented-reality system 500 to determine relative positioning of each acoustic transducer 520 in the microphone array.

In some examples, the augmented-reality system 500 may include or be connected to an external device (e.g., a paired device), such as the neckband 505. The neckband 505 generally represents any type or form of paired device. Thus, the following discussion of the neckband 505 may also apply to various other paired devices, such as charging cases, smart watches, smart phones, wrist bands, other wearable devices, hand-held controllers, tablet computers, laptop computers, other external compute devices, etc.

As shown, the neckband 505 may be coupled to the eyewear device 502 via one or more connectors. The connectors may be wired or wireless and may include electrical and/or non-electrical (e.g., structural) components. In some cases, the eyewear device 502 and the neckband 505 may operate independently without any wired or wireless connection between them. While FIG. 5 illustrates the components of the eyewear device 502 and the neckband 505 in example locations on the eyewear device 502 and the neckband 505, the components may be located elsewhere and/or distributed differently on the eyewear device 502 and/or the neckband 505. In some examples, the components of the eyewear device 502 and the neckband 505 may be located on one or more additional peripheral devices paired with the eyewear device 502, the neckband 505, or some combination thereof.

Pairing external devices, such as the neckband 505, with augmented-reality eyewear devices may enable the eyewear devices to achieve the form factor of a pair of glasses while still providing sufficient battery and computation power for expanded capabilities. Some or all of the battery power, computational resources, and/or additional features of the augmented-reality system 500 may be provided by a paired device or shared between a paired device and an eyewear device, thus reducing the weight, heat profile, and form factor of the eyewear device overall while still retaining desired functionality. For example, the neckband 505 may allow components that would otherwise be included on an eyewear device to be included in the neckband 505 since users may tolerate a heavier weight load on their shoulders than they would tolerate on their heads. The neckband 505 may also have a larger surface area over which to diffuse and disperse heat to the ambient environment. Thus, the neckband 505 may allow for greater battery and computation capacity than might otherwise have been possible on a standalone eyewear device. Since weight carried in the neckband 505 may be less invasive to a user than weight carried in the eyewear device 502, a user may tolerate wearing a lighter eyewear device and carrying or wearing the paired device for greater lengths of time than a user would tolerate wearing a heavy standalone eyewear device, thereby enabling users to more fully incorporate artificial-reality environments into their day-to-day activities.

The neckband 505 may be communicatively coupled with the eyewear device 502 and/or to other devices. These other devices may provide certain functions (e.g., tracking, localizing, depth mapping, processing, storage, etc.) to the augmented-reality system 500. In the example of FIG. 5, the neckband 505 may include two acoustic transducers (e.g., 520(I) and 520(J)) that are part of the microphone array (or potentially form their own microphone subarray). The neckband 505 may also include a controller 525 and a power source 535.

The acoustic transducers 520(I) and 520(J) of the neckband 505 may be configured to detect sound and convert the detected sound into an electronic format (analog or digital). In the example of FIG. 5, the acoustic transducers 520(I) and 520(J) may be positioned on the neckband 505, thereby increasing the distance between the neckband acoustic transducers 520(I) and 520(J) and other acoustic transducers 520 positioned on the eyewear device 502. In some cases, increasing the distance between the acoustic transducers 520 of the microphone array may improve the accuracy of beamforming performed via the microphone array. For example, if a sound is detected by the acoustic transducers 520(C) and 520(D) and the distance between the acoustic transducers 520(C) and 520(D) is greater than, e.g., the distance between the acoustic transducers 520(D) and 520(E), the determined source location of the detected sound may be more accurate than if the sound had been detected by the acoustic transducers 520(D) and 520(E).

The controller 525 of the neckband 505 may process information generated by the sensors on the neckband 505 and/or the augmented-reality system 500. For example, the controller 525 may process information from the microphone array that describes sounds detected by the microphone array. For each detected sound, the controller 525 may perform a direction-of-arrival (DOA) estimation to estimate a direction from which the detected sound arrived at the microphone array. As the microphone array detects sounds, the controller 525 may populate an audio data set with the information. In examples in which the augmented-reality system 500 includes an inertial measurement unit, the controller 525 may compute all inertial and spatial calculations from the IMU located on the eyewear device 502. A connector may convey information between the augmented-reality system 500 and the neckband 505 and between the augmented-reality system 500 and the controller 525. The information may be in the form of optical data, electrical data, wireless data, or any other transmittable data form. Moving the processing of information generated by the augmented-reality system 500 to the neckband 505 may reduce weight and heat in the eyewear device 502, making it more comfortable to the user.

The power source 535 in the neckband 505 may provide power to the eyewear device 502 and/or to the neckband 505. The power source 535 may include, without limitation, lithium-ion batteries, lithium-polymer batteries, primary lithium batteries, alkaline batteries, or any other form of power storage. In some cases, the power source 535 may be a wired power source. Including the power source 535 on the neckband 505 instead of on the eyewear device 502 may help better distribute the weight and heat generated by the power source 535.

As noted, some artificial-reality systems may, instead of blending an artificial reality with actual reality, substantially replace one or more of a user's sensory perceptions of the real world with a virtual experience. One example of this type of system is a head-worn display system, such as virtual-reality system 600 in FIG. 6, that mostly or completely covers a user's field of view. The virtual-reality system 600 may include a front rigid body 602 and a band 604 shaped to fit around a user's head. The virtual-reality system 600 may also include output audio transducers 606(A) and 606(B). Furthermore, while not shown in FIG. 6, the front rigid body 602 may include one or more electronic elements, including one or more electronic displays, one or more inertial measurement units (IMUs), one or more tracking emitters or detectors, and/or any other suitable device or system for creating an artificial-reality experience.

Artificial-reality systems may include a variety of types of visual feedback mechanisms. For example, display devices in the augmented-reality system 500 and/or the virtual-reality system 600 may include one or more liquid crystal displays (LCDs), light emitting diode (LED) displays, microLED displays, organic LED (OLED) displays, digital light project (DLP) micro-displays, liquid crystal on silicon (LCoS) micro-displays, and/or any other suitable type of display screen. These artificial-reality systems may include a single display screen for both eyes or may provide a display screen for each eye, which may allow for additional flexibility for varifocal adjustments or for correcting a user's refractive error. Some of these artificial-reality systems may also include optical subsystems having one or more lenses (e.g., concave or convex lenses, Fresnel lenses, adjustable liquid lenses, etc.) through which a user may view a display screen. These optical subsystems may serve a variety of purposes, including to collimate (e.g., make an object appear at a greater distance than its physical distance), to magnify (e.g., make an object appear larger than its actual size), and/or to relay (to, e.g., the viewer's eyes) light. These optical subsystems may be used in a non-pupil-forming architecture (such as a single lens configuration that directly collimates light but results in so-called pincushion distortion) and/or a pupil-forming architecture (such as a multi-lens configuration that produces so-called barrel distortion to nullify pincushion distortion).

In addition to or instead of using display screens, some of the artificial-reality systems described herein may include one or more projection systems. For example, display devices in the augmented-reality system 500 and/or the virtual-reality system 600 may include micro-LED projectors that project light (using, e.g., a waveguide) into display devices, such as clear combiner lenses that allow ambient light to pass through. The display devices may refract the projected light toward a user's pupil and may enable a user to simultaneously view both artificial-reality content and the real world. The display devices may accomplish this using any of a variety of different optical components, including waveguide components (e.g., holographic, planar, diffractive, polarized, and/or reflective waveguide elements), light-manipulation surfaces and elements (such as diffractive, reflective, and refractive elements and gratings), coupling elements, etc. Artificial-reality systems may also be configured with any other suitable type or form of image projection system, such as retinal projectors used in virtual retina displays.

The artificial-reality systems described herein may also include various types of computer vision components and subsystems. For example, the augmented-reality system 500 and/or the virtual-reality system 600 may include one or more optical sensors, such as two-dimensional (2D) or 3D cameras, structured light transmitters and detectors, time-of-flight depth sensors, single-beam or sweeping laser rangefinders, 3D LiDAR sensors, and/or any other suitable type or form of optical sensor. An artificial-reality system may process data from one or more of these sensors to identify a location of a user, to map the real world, to provide a user with context about real-world surroundings, and/or to perform a variety of other functions.

The artificial-reality systems described herein may also include one or more input and/or output audio transducers. Output audio transducers may include voice coil speakers, ribbon speakers, electrostatic speakers, piezoelectric speakers, bone conduction transducers, cartilage conduction transducers, tragus-vibration transducers, and/or any other suitable type or form of audio transducer. Similarly, input audio transducers may include condenser microphones, dynamic microphones, ribbon microphones, and/or any other type or form of input transducer. In some examples, a single transducer may be used for both audio input and audio output.

In some examples, the artificial-reality systems described herein may also include tactile (i.e., haptic) feedback systems, which may be incorporated into headwear, gloves, body suits, handheld controllers, environmental devices (e.g., chairs, floormats, etc.), and/or any other type of device or system. Haptic feedback systems may provide various types of cutaneous feedback, including vibration, force, traction, texture, and/or temperature. Haptic feedback systems may also provide various types of kinesthetic feedback, such as motion and compliance. Haptic feedback may be implemented using motors, piezoelectric actuators, fluidic systems, and/or a variety of other types of feedback mechanisms. Haptic feedback systems may be implemented independent of other artificial-reality devices, within other artificial-reality devices, and/or in conjunction with other artificial-reality devices.

By providing haptic sensations, audible content, and/or visual content, artificial-reality systems may create an entire virtual experience or enhance a user's real-world experience in a variety of contexts and environments. For instance, artificial-reality systems may assist or extend a user's perception, memory, or cognition within a particular environment. Some systems may enhance a user's interactions with other people in the real world or may enable more immersive interactions with other people in a virtual world. Artificial-reality systems may also be used for educational purposes (e.g., for teaching or training in schools, hospitals, government organizations, military organizations, business enterprises, etc.), entertainment purposes (e.g., for playing video games, listening to music, watching video content, etc.), and/or for accessibility purposes (e.g., as hearing aids, visual aids, etc.). The examples disclosed herein may enable or enhance a user's artificial-reality experience in one or more of these contexts and environments and/or in other contexts and environments.

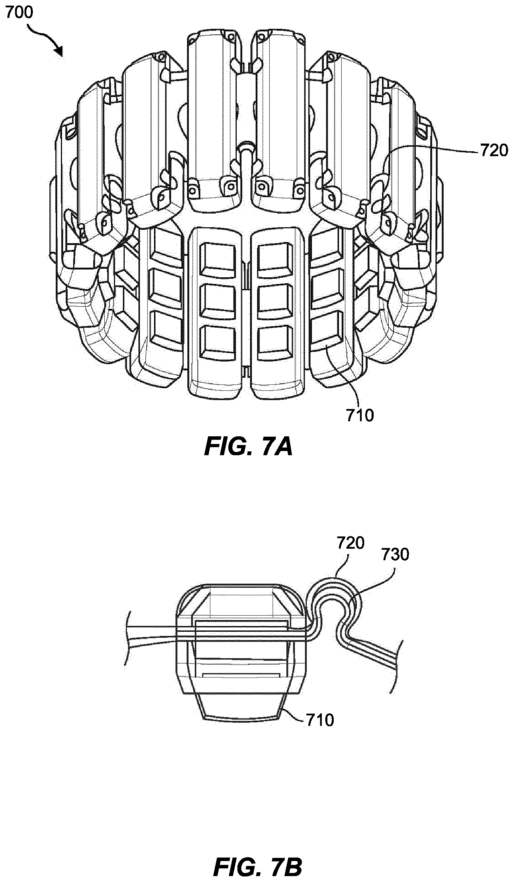

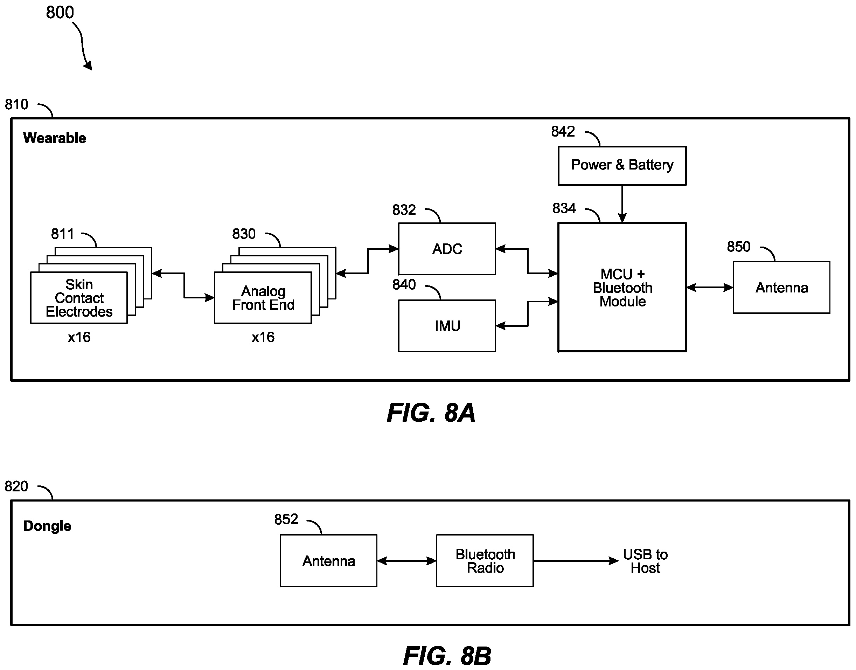

FIG. 7A illustrates an example human-machine interface (also referred to herein as an EMG control interface) configured to be worn around a user's lower arm or wrist as a wearable system 700. In this example, the wearable system 700 may include sixteen neuromuscular sensors 710 (e.g., EMG sensors) arranged circumferentially around an elastic band 720 with an interior surface 730 configured to contact a user's skin. However, any suitable number of neuromuscular sensors may be used. The number and arrangement of neuromuscular sensors may depend on the particular application for which the wearable device is used. For example, a wearable armband or wristband can be used to generate control information for controlling an augmented reality system, a robot, controlling a vehicle, scrolling through text, controlling a virtual avatar, or any other suitable control task. As shown, the sensors may be coupled together using flexible electronics incorporated into the wireless device. FIG. 7B illustrates a cross-sectional view through one of the sensors of the wearable device shown in FIG. 7A. In some examples, the output of one or more of the sensing components can be optionally processed using hardware signal processing circuitry (e.g., to perform amplification, filtering, and/or rectification). In other examples, at least some signal processing of the output of the sensing components can be performed in software. Thus, signal processing of signals sampled by the sensors can be performed in hardware, software, or by any suitable combination of hardware and software, as aspects of the technology described herein are not limited in this respect. A non-limiting example of a signal processing chain used to process recorded data from the sensors 710 is discussed in more detail below with reference to FIGS. 8A and 8B.

FIGS. 8A and 8B illustrate an example schematic diagram with internal components of a wearable system with EMG sensors. As shown, the wearable system may include a wearable portion 810 (FIG. 8A) and a dongle portion 820 (FIG. 8B) in communication with the wearable portion 810 (e.g., via BLUETOOTH or another suitable wireless communication technology). As shown in FIG. 8A, the wearable portion 810 may include skin contact electrodes 811, examples of which are described in connection with FIGS. 7A and 7B. The output of the skin contact electrodes 811 may be provided to an analog front end 830, which may be configured to perform analog processing (e.g., amplification, noise reduction, filtering, etc.) on the recorded signals. The processed analog signals may then be provided to an analog-to-digital converter 832, which may convert the analog signals to digital signals that can be processed by one or more computer processors. An example of a computer processor that may be used in accordance with some examples is a microcontroller (MCU) 834, illustrated in FIG. 8A. As shown, the MCU 834 may also include inputs from other sensors (e.g., an IMU sensor 840), and a power and battery module 842. The output of the processing performed by the MCU 834 may be provided to an antenna 850 for transmission to the dongle portion 820 shown in FIG. 8B.

The dongle portion 820 may include an antenna 852, which may be configured to communicate with the antenna 850 included as part of the wearable portion 810. Communication between the antennas 850 and 852 may occur using any suitable wireless technology and protocol, non-limiting examples of which include radiofrequency signaling and BLUETOOTH ^{®}. As shown, the signals received by the antenna 852 of the dongle portion 820 may be provided to a host computer for further processing, display, and/or for effecting control of a particular physical or virtual object or objects.

Although the examples provided with reference to FIGS. 7A-7B and FIGS. 8A-8B are discussed in the context of interfaces with EMG sensors, the techniques described herein for reducing electromagnetic interference can also be implemented in wearable interfaces with other types of sensors including, but not limited to, mechanomyography (MMG) sensors, sonomyography (SMG) sensors, and electrical impedance tomography (EIT) sensors. The techniques described herein for reducing electromagnetic interference can also be implemented in wearable interfaces that communicate with computer hosts through wires and cables (e.g., USB cables, optical fiber cables, etc.).

The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed. The various example methods described and/or illustrated herein may also omit one or more of the steps described or illustrated herein or include additional steps in addition to those disclosed.

The preceding description has been provided to enable others skilled in the art to best utilize various aspects of the examples disclosed herein. This example description is not intended to be exhaustive or to be limited to any precise form disclosed. Many modifications and variations are possible without departing from the scope of the present disclosure. The examples disclosed herein should be considered in all respects illustrative and not restrictive. Reference should be made to any claims appended hereto and their equivalents in determining the scope of the present disclosure.

Unless otherwise noted, the terms "connected to" and "coupled to" (and their derivatives), as used in the specification and/or claims, are to be construed as permitting both direct and indirect (i.e., via other elements or components) connection. In addition, the terms "a" or "an," as used in the specification and/or claims, are to be construed as meaning "at least one of." Finally, for ease of use, the terms "including" and "having" (and their derivatives), as used in the specification and/or claims, are interchangeable with and have the same meaning as the word "comprising."

## Claims

1. An electronic device, comprising:
a non-conductive housing component;
a conductive seed material formed over an external surface of the non-conductive housing component;
a bulk conductive contact material formed over the conductive seed material; and
an electronic component connected to the bulk conductive contact material and the conductive seed material.

2. The electronic device of claim 1, further comprising a conductive via passing through the non-conductive housing component and connecting the bulk conductive contact material and the conductive seed material to the electronic component.

3. The electronic device of claim 1 or claim 2, further comprising a conductive connection, comprising at least one of: a conductive post through the non-conductive housing component; a conductive plating on a surface of the non-conductive housing component; or a conductive strip molded in the non-conductive housing component, the conductive connection connecting the bulk conductive contact material and the conductive seed material to the electronic component.

4. The electronic device of any preceding claim, wherein the electronic component comprises a battery and the bulk conductive contact material comprises charging contacts for the battery.

5. The electronic device of any preceding claim, further comprising at least one conductive coating material over at least a portion of the bulk conductive contact material, preferably wherein the at least one conductive coating material comprises at least one of: nitride; diamond-like carbon; chromium; palladium; gold; platinum; silver; or rhodium.

6. The electronic device of any preceding claim, wherein the electronic component comprises a sensing circuit and the bulk conductive contact material comprises at least one sensor electrode for the sensing circuit;
preferably wherein the sensing circuit comprises an electromyography sensing circuit.

7. The electronic device of any preceding claim, wherein the bulk conductive contact material comprises an electrode pair including two electrodes.

8. A method of forming contacts for an electronic device, the method comprising:
forming a conductive seed material over a non-conductive housing component of the electronic device;
forming a bulk conductive contact material over the conductive seed material; and
connecting the bulk conductive contact material and the conductive seed material to an electronic component.

9. The method of claim 8, wherein connecting the bulk conductive contact material and the conductive seed material to the electronic component comprises:
molding at least one of a conductive post or a conductive strip in the non-conductive housing component;
forming the conductive seed material in electrical contact with the conductive post or the conductive strip; and
electrically connecting the conductive post or the conductive strip to the electronic component; or comprises:
forming a conductive via through at least a portion of the non-conductive housing component;
forming the conductive seed material in electrical contact with the conductive via; and
electrically connecting the conductive via to the electronic component.

10. The method of claim 8 or 9, wherein the electronic component comprises a battery.

11. The method of an of claims 8 to 10, wherein the electronic component comprises a sensing circuit; preferably wherein the sensing circuit comprises an electromyography sensing circuit.

12. The method of any of claims 8 to 11, further comprising coating at least a portion of the bulk conductive contact material with at least one conductive coating material, preferably wherein the at least one conductive coating material comprises at least one of: nitride; diamond-like carbon; chromium; palladium; gold; platinum; silver; or rhodium.

13. The method of any of claims 8 to 12, wherein forming the conductive seed material over the non-conductive housing component comprises forming at least one of: copper; nickel, titanium, chromium; or gold over the non-conductive housing component.

14. The method of any of claims 8 to 13, further comprising etching a surface of the non-conductive housing component, wherein forming the conductive seed material comprises forming the conductive seed material on the etched surface of the non-conductive housing component.

15. The method of any of claims 8 to 14, wherein forming the bulk conductive contact material over the conductive seed material comprises:
forming at least one of: copper; nickel; or silver over the conductive seed material; and/or
forming the bulk conductive contact material to a thickness between 1 µm and 30 µm.
